(19) Europäisches Patentamt
European Patent Office
Office européen des brevets

(11) Veröffentlichungsnummer : **0 031 023**
**B1**

(12) **EUROPÄISCHE PATENTSCHRIFT**

(45) Veröffentlichungstag der Patentschrift : **15.12.82**

(21) Anmeldenummer : **80107006.1**

(22) Anmeldetag : **13.11.80**

(51) Int. Cl.$^3$ : **C 07 C 69/74**, C 07 D309/22, C 07 D309/10, C 07 D335/02, C 07 C 69/743, A 01 N 53/00

# (54) Cyclopropancarbonsäureester, Verfahren zu ihrer Herstellung und ihre Verwendung.

(30) Priorität : **22.12.79 DE 2951939**

(43) Veröffentlichungstag der Anmeldung : **01.07.81 (Patentblatt 81/26)**

(45) Bekanntmachung des Hinweises auf die Patenterteilung : **15.12.82 Patentblatt 82/50**

(84) Benannte Vertragsstaaten : **CH FR GB IT LI**

(56) Entgegenhaltungen :
**EP A 0 000 506**
**FR A 2 226 383**

(73) Patentinhaber : **Degussa Aktiengesellschaft**
**Weissfrauenstrasse 9**
**D-6000 Frankfurt am Main 1 (DE)**

(72) Erfinder : **Schwarze, Werner, Dr.**
**Leerbachstrasse 17**
**D-6000 Frankfurt (Main) (DE)**
Erfinder : **Kleemann, Axel, Dr.**
**Greifenhagenstrasse 9**
**D-6450 Hanau 9 (DE)**

**EP 0 031 023 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Cyclopropancarbonsäureester, Verfahren zu ihrer Herstellung und ihre Verwendung.

Die vorliegende Erfindung betrifft Cyclopropancarbonsäureester, Verfahren zu ihrer Herstellung und ihre Verwendung in der Schädlingsbekämpfung.

Die erfindungsgemässen Cyclopropancarbonsäureester entsprechen der Formel

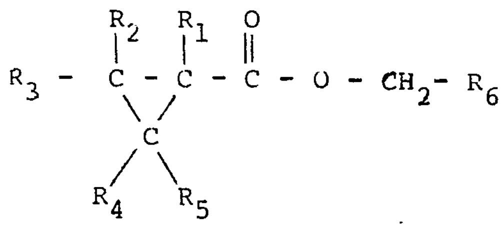

(I)

worin $R_1$, $R_2$, $R_3$, $R_4$ und $R_5$ je ein Wasserstoffatom oder einen Alkylrest, $R_6$ die Reste

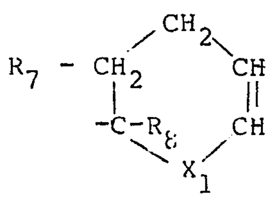

,

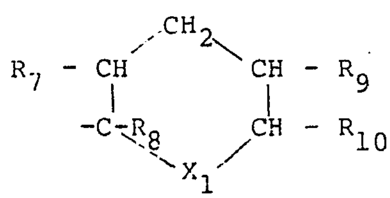

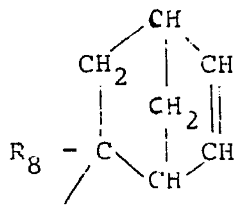

$R_7$ ein Wasserstoffatom, den Methyl- oder Phenyl-Rest, $R_8$ ein Wasserstoffatom, den Methyl- oder den

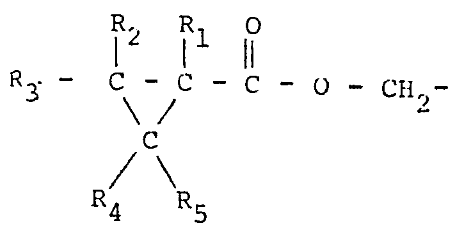

Rest,

in welchem $R_1$ bis $R_5$ für die oben angegebenen Bedeutungen stehen, $X_1$ für ein Sauerstoffatom, ein Schwefelatom oder für die $-CH_2$-Gruppe steht, und $R_9$ und $R_{10}$ je ein Wasserstoffatom oder ein Halogenatom bedeuten. Unter Halogenatomen sind Fluor, Chlor, Brom oder Jod, insbesondere aber Chlor oder Brom, zu verstehen.

Die bei $R_1$ bis $R_5$ in Frage kommenden Alkylgruppen können geradkettig oder verzweigt sein und haben in der Kette vorzugsweise 1 bis 5 Kohlenstoffatome. Beispiele solcher Gruppen sind : Methyl, Äthyl, n-Propyl, Isopropyl, n-, i-, sek.-, tert.-Butyl, n-Pentyl und dessen Isomere.

Wegen ihrer Wirkung bevorzugt sind Verbindungen der Formel I, worin $R_1$ ein Wasserstoffatom oder einen $C_1$-$C_5$-Alkylrest, $R_2$, $R_3$, $R_4$ und $R_5$ je ein Wasserstoffatom oder den Methylrest, $R_6$ die Reste

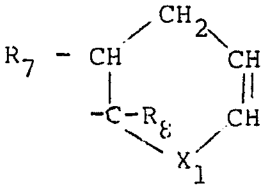

oder

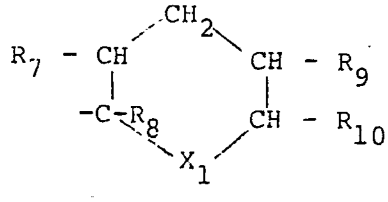

,

$R_7$ und $R_8$ je ein Wasserstoffatom, $X_1$ ein Sauerstoffatom oder den $-CH_2$-Rest und $R_9$ und $R_{10}$ je ein Wasserstoff-, Chlor- oder Bromatom bedeuten.

Insbesondere bevorzugt sind Verbindungen der Formel I, worin $R_1$, $R_2$, $R_3$, $R_4$ und $R_5$ je ein Wasserstoffatom, $R_6$ die Reste

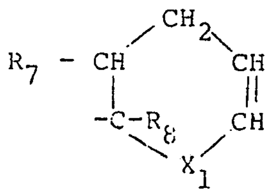

oder

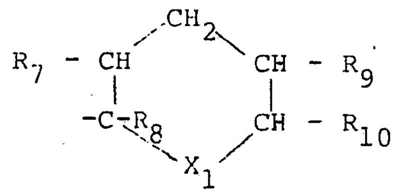

$R_7$ und $R_8$ je ein Wasserstoffatom, $X_1$ ein Sauerstoffatom oder die $—CH_2$-Gruppe und $R_9$ und $R_{10}$ je ein Wasserstoff-, Chlor- oder Bromatom bedeuten.

Die Verbindungen der Formel I können nach an sich bekannten Methoden zum Beispiel wie folgt hergestellt werden :

1)

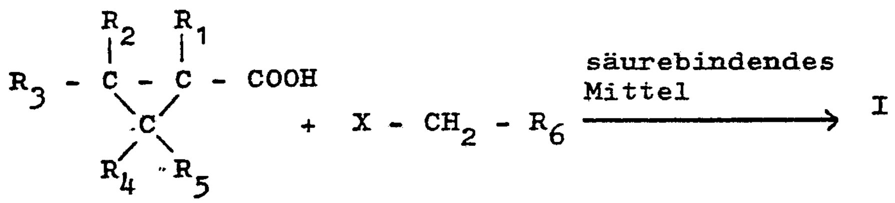


(II) (III)

2)

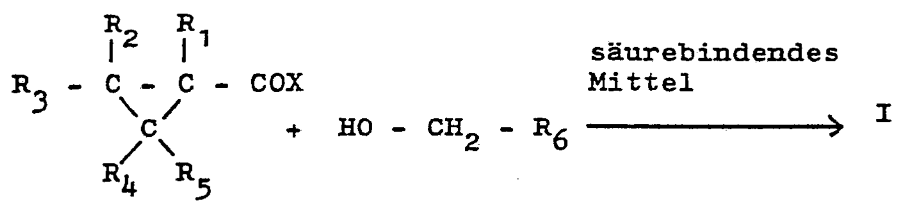


(IV) (V)

3)

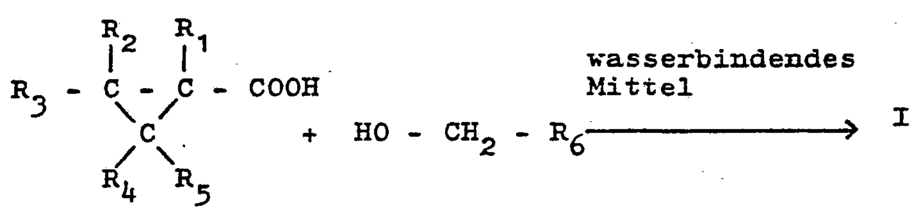


(II) (V)

4)

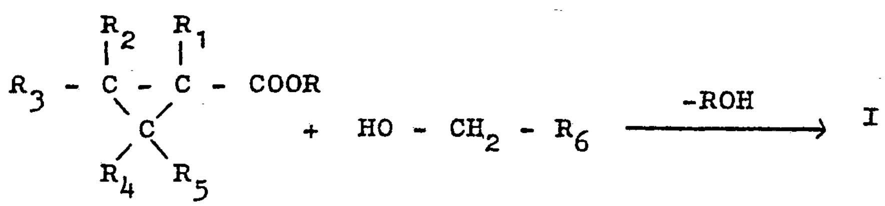


(VI) (V)

In den Formeln II bis VI haben $R_1$, $R_2$, $R_3$, $R_4$, $R_5$ und $R_6$ die für die Formel I angegebene Bedeutung.

In den Formeln III und IV steht X für ein Halogenatom, insbesondere Chlor oder Brom, und in der Formel VI steht R für einen $C_1$-$C_4$-Alkylrest, insbesondere für den Methyl- oder Äthylrest. Als säurebindendes Mittel für die Verfahren 1 und 2 kommen insbesondere tertiäre Amine, wie Trialkylamine und Pyridin, ferner Hydroxide, Oxide, Carbonate und Bicarbonate von Alkali- und Erdalkalimetallen, sowie Alkalimetallalkoholate, wie zum Beispiel Kalium-t-butylat und Natriummethylat, in Betracht. Als wasserbindendes Mittel für das Verfahren 3 kann zum Beispiel Bicyclohexylcarbodiimid verwendet werden. Die Verfahren 1 bis 4 werden bei einer Reaktionstemperatur zwischen – 10 und 120 °C, meist zwischen 20 und 80 °C, bei normalem oder erhöhtem Druck, und vorzugsweise in einem inerten Lösungs- oder Verdünnungsmittel, durchgeführt. Als Lösungs- oder Verdünnungsmittel eignen sich zum Beispiel

Äther und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ester der gesättigten Dibrom- oder Dichlor-hexahydrobenzylalkohole können auch auf andere Art und Weise hergestellt werden, zum Beispiel dadurch, dass man an die ungesättigten Tetrahydrobenzylalkoholester nachträglich Brom bzw. Chlor addiert. Gleiches gilt für die ungesättigten Pyran- bzw. Thiapyranalkohole.

Die Ausgangsstoffe der Formeln II bis VI sind bekannt und können nach bekannten Methoden hergestellt werden.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken zum Beispiel der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung der Eier, Larven und Imagines von pflanzenschädigenden Insekten, insbesondere pflanzenschädigenden Frassinsekten, in Zier- und Nutzpflanzen, insbesondere in Baumwollkulturen (zum Beispiel gegen Spodoptera littoralis und Heliothis virescens) und Gemüsekulturen (zum Beispiel gegen Leptinotarsa decemlineata und Myzus persicae) und Obstkulturen (zum Beispiel gegen Laspeyresia pomonella).

Wirkstoffe der Formel I zeigen auch eine sehr günstige Wirkung gegen Fliegen, wie zum Beispiel Musca domestica, und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Verbindungen der Formel I können für sich allein oder zusammen mit geeigneten Trägern und/oder Zuschlagstoffen eingesetzt werden. Geeignete Träger- und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergieroder Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate); Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt zwischen 0,1 bis 95 %, dabei ist zu erwähnen, dass bei der Applikation aus dem Flugzeug oder mittels anderer geeigneter Applikationsgeräte Konzentrationen bis zu 99,5 % oder sogar reiner Wirkstoff eingesetzt werden können. Die Wirkstoffe der Formel I können beispielsweise wie folgt formuliert werden (Teile bedeuten Gewichtsteile):

Stäubemittel: Zur Herstellung eines a) 5 %igen und b) 2 %igen Stäubemittels werden die folgenden Stoffe verwendet:

a) 5 Teile Wirkstoff
95 Teile Talkum;

b) 2 Teile Wirkstoff
1 Teil hochdisperse Kieselsäure
97 Teile Talkum.

Der Wirkstoff wird mit den Trägerstoffen vermischt und vermahlen.

Granulat: Zur Herstellung eines 5 %igen Granulates werden die folgenden Stoffe verwendet:

5 Teile Wirkstoff

0,25 Teile epoxydiertes Pflanzenöl
0,25 Teile Cetylpolyglykoläther
3,50 Teile Polyäthylenglykol
91 Teile Kaolin (Korngrösse 0,3-0,8 mm).

Die Aktivsubstanz wird mit epoxydiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum eingedampft.

Spritzpulver : Zur Herstellung eines a) 40 %igen, b) und c) 25 %igen, d) 10 %igen Spritzpulvers werden folgende Bestandteile verwendet :

a) 40 Teile Wirkstoff
5 Teile Ligninsulfonsäure-Natriumsalz
1 Teil Dibutylnaphthalinsulfonsäure-Natriumsalz
54 Teile Kieselsäure ;

b) 25 Teile Wirkstoff
4,5 Teile Calcium-Ligninsulfonat
1,9 Teile Champagne-Kreide/Hydroxyäthylcellulose-Gemisch (1 : 1)
1,5 Teile Natrium-dibutyl-naphthalinsulfonat
19,5 Teile Kieselsäure
19,5 Teile Champagne Kreide
28,1 Teile Kaolin ;

c) 25 Teile Wirkstoff
2,5 Teile Isooctylphenoxy-polyäthylen-äthanol
1,7 Teile Champagne-Kreide/Hydroxyäthylcellulose-Gemisch (1 : 1)
8,3 Teile Natriumaluminiumsilikat
16,5 Teile Kieselgur
46 Teile Kaolin ;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate : Zur Herstellung eines a) 10 %igen, b) 25 %igen und c) 50 %igen emulgierbaren Konzentrates werden folgende Stoffe verwendet :

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylfromamid
43,2 Teile Xylol ;

b) 25 Teile Wirkstoff
2,5 Teile epoxydierte Pflanzenöl
10 Teile eines Alkylarylsulfonat/Fettalkoholpolyglykoläther-Gemisches
5 Teile Dimethylformamid
57,5 Teile Xylol ;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentraten können durch Verdünnen mit Wasser Emulsionen jeder gewünschten Konzentration hergestellt werden.

Sprühmittel : Zur Herstellung eines a) 5 %igen und b) 95 %igen Sprühmittels werden die folgenden Bestandteile verwendet :

a) 5 Teile Wirkstoff
1 Teil epoxydiertes Pflanzenöl
94 Teile Benzin (Siedegrenzen 160-190 °C) ;

b) 95 Teile Wirkstoff
5 Teile epoxydiertes Pflanzenöl

## Beispiel 1

86 g Cyclopropancarbonsäure (= 1 Mol) und 117,6 g Tetrahydrobenzylalkohol-1,2,3,6 (= 1,05 Mol) vermischt man mit 400 ml Toluol, gibt 2 g p-Toluolsulfonsäure hinzu und bringt die Mischung zum Sieden. Das bei der Veresterung gebildete Wasser wird azeotrop ausgekreist. Nach 2 Stunden sind ca. 18 ml Wasser abgeschieden. Man kühlt ab, wäscht mit Wasser (+ wenig $NaHCO_3$) neutral und engt die Lösung am Rotationsverdampfer ein. Der Rest wird an einer 40 cm-Vigreux-Kolonne im Vakuum rektifiziert.

Bei $Kp_{10}$ 122 °C destilliert der Cyclopropancarbonsäuretetrahydrobenzylester, Menge : 148.4 g, entsprechend 82,6 % d. Th.

Farblose Flüssigkeit.
Analyse : $C_{11}H_{16}O_2$ (Mol-Gew. 180)
ber. C 73,3 H 8,88
gef. C 73,2 H 8,6

## Beispiel 2

180 g Cyclopropancarbonsäuretetrahydrobenzylalkohol (1,2,3,6)-Ester (= 1 Mol) löst man in 1 l Tetrachlorkohlenstoff. Man kühlt auf 0 °C ab. Nun lässt man eine Lösung von 160 g Brom in 500 ml Tetrachlorkohlenstoff zulaufen, wobei man darauf achtet, dass die Reaktionstemperatur + 5 °C nicht übersteigt. Die Addition erfolgt momentan. Anschliessend wird mit Wasser + $NaHCO_3$ neutral gewaschen. Die Lösung wird am Rotationsverdampfer eingeengt und anschliessend im Vakuum destilliert.

Bei $Kp_{2,5}$ 164-166 °C destilliert der Cyclopropancarbonsäureester des 4,5-Dibrom-hexahydrobenzylalkohols-1.

Farblose Flüssigkeit, Menge : 282,8 g = 83.2 % d. Th.
Analyse : $C_{11}H_{16}Br_2O_2$ (Mol-Gew. 340)
ber. C 38,8 H 4,7 Br 47,1
gef. C 38,5 H 4,7 Br 48,1

## Beispiel 3

28,4 g 3-Cyclohexen-1,1-dimethanol (= 0,2 Mol) löst man in 150 ml Acetonitril, dazu gibt man 17,4 g Pyridin (= ca. 0,22 Mol). Die Mischung wird auf 0 °C gekühlt. Bei dieser Temperatur tropft man langsam 22 g (= ca. 0,21 Mol) Cyclopropancarbonsäurechlorid hinzu, anschliessend erwärmt man langsam auf Zimmertemperatur (20 °C). Nach 2 Stunden giesst man auf Eis. Das sich abscheidende Öl wird mit Methylenchlorid ausgeschüttelt. Man dampft die organische Phase im Vakuum ein. Zurück bleiben weisse Kristalle, die mit Petroläther digeriert und abgenutscht werden. F. 76-77 °C, Menge : 47,8 g = 86 % d. Th., bezogen auf 3-Cyclohexen-1,1-dimethanol-bis-cyclopropancarbonsäureester.

Analyse : $C_{16}H_{22}O_4$ (Mol-Gew. 278)
ber. C 69,1 H 7,9
gef. C 69,3 H 7,9

## Beispiel 4

128 g 2-Methylcyclopropancarbonsäureäthylester (= 1 Mol) und 117,6 g Tetrahydrobenzylalkohol-1,2,3,6 (= 1,05 Mol) gibt man in 1 l Toluol. Dann setzt man 2 g Natriumäthylat hinzu und erhitzt zum Sieden. Über eine 40 cm-Vigreux-Kolonne mit Dephlegmator wird das Azeotrop Toluol-Äthanol langsam abdestilliert. Die Umesterung ist in 6 Stunden beendet.

Man lässt erkalten und wäscht dann die Reaktionsmischung neutral, anschliessend wird das Lösungsmittel am Rotationsverdampfer im Vakuum abgezogen. Der Rest wird im Vakuum rektifiziert. Bei $Kp_{15}$ 128-130 °C destilliert der 2-Methylcyclopropancarbonsäureester des 1,2,3,6-Tetrahydrobenzylalkohols, 151 g = 77,9 % d. Th.

Analyse : $C_{12}H_{18}O_2$ (Mol-Gew. 194)
ber. C 74,3 H 9,3
gef. C 74,1 H 9,2

Beispiel 5

46,2 g 2,2-Dimethylol-bicyclo-[2,2,1]-hepten-5- (= 0,3 Mol) und Cyclopropancarbonsäure (0,3 Mol) werden mit Bicyclohexylcarbodiimid nach der Methode von A. Buzas et al., Compt. rend. *255*, 945 (1962), umgesetzt.

Man erhält 30,4 g Diester, $Kp_{0,6}$ 152-155 °C, entsprechend einer Ausbeute von 35,2 % d. Th.

Analyse : $C_{17}H_{22}O_4$ (Mol-Gew. 288)

ber. C 71,0 H 7,7

gef. C 70,8 H 7,8

Auf analoge Weise wurden auch folgende Verbindungen hergestellt :

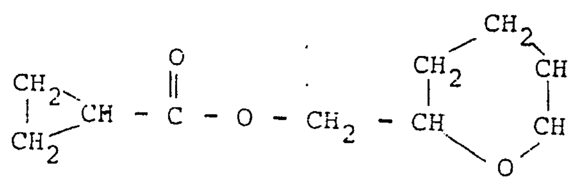

Sdp. : 122 °C/10 mm/Hg

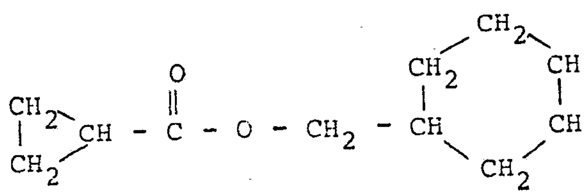

Sdp. : 122 °C/10 mm/Hg

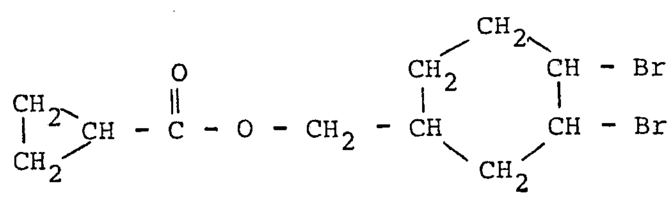

Sdp. : 164-166 °C/2,5 mm/Hg

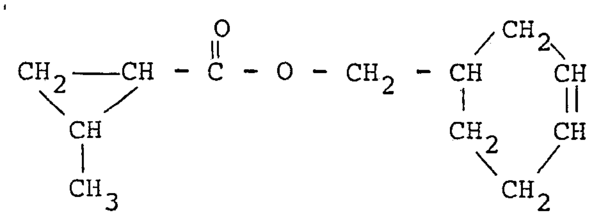

Sdp. : 128-130 °C/15 mm/Hg

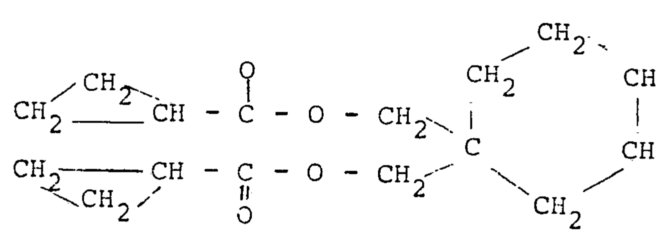

Sdp. : 76-77 °C

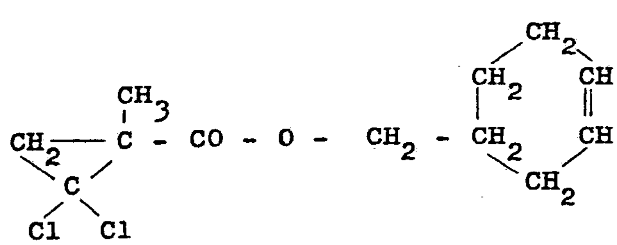

Sdp. : 162-163 °C/15 mm Hg

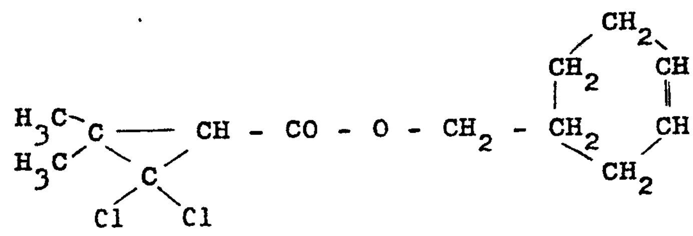

Sdp. : 168-170 °C/15 mm Hg

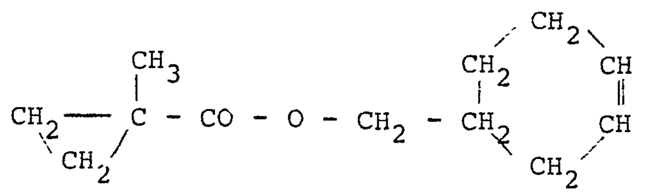

Sdp. : 123-125 °C/15 mm Hg

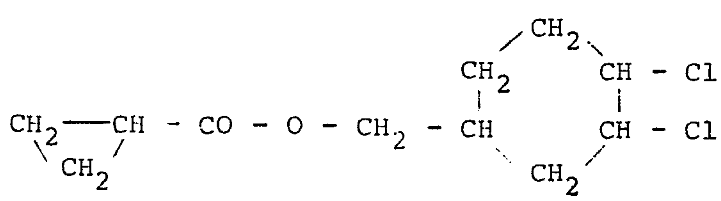

Sdp. : 112-113 °C/15 mm Hg

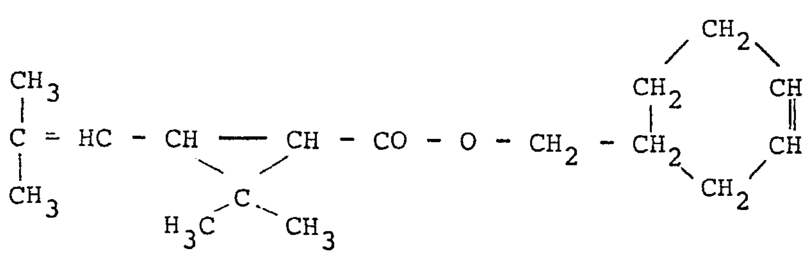

Sdp. : 175-176 °C/15 mm Hg

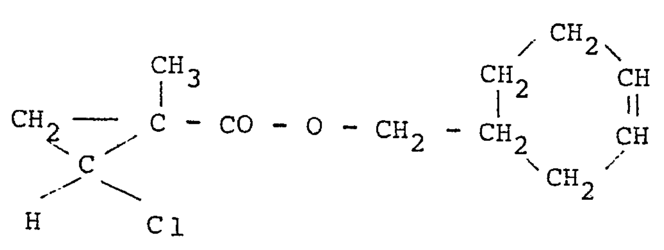

Sdp. : 88 °C/0,4 mm Hg

Beispiel 6

Insektizide Frassgift-Wirkung : Spodoptera littoralis, Dysdercus fasciatus und Heliothis virescens

Baumwollpflanzen wurden mit einer wässrigen Emulsion enthaltend 0,05 % der zu prüfenden Verbindung (erhalten aus einem 10 %igen emulgierbaren Konzentrat) besprüht.

Nach dem Antrocknen des Belages wurden die Pflanzen je mit Larven der Spezies Spodoptera littoralis (L3-Stadium), Dysdercus fasciatus (L4) oder Heliothis virescens (L3) besetzt. Man verwendete pro Versuchsverbindung und pro Test-Spezies zwei Pflanzen und eine Auswertung der erzielten Abtötungsrate erfolgte nach 2, 4, 24 und 48 Stunden. Der Versuch wurde bei 24 °C und bei 60 % relativer

Luftfeuchtigkeit durchgeführt.

Die Verbindungen gemäss Beispiel 5 zeigten im obigen Versuch eine gute Wirkung gegen Larven der Spezies Spodoptera littoralis, Dysdercus fasciatus und Heliothis virescens.

Beispiel 7

Insektizide Frassgift-Wirkung : Leptinotarsa decemlineata

Bei gleicher Arbeitsweise unter Verwendung von Larven der Spezies Leptinotarsa decemlineata (L3) und Kartoffelpflanzen anstelle von Baumwollpflanzen wurde die im Beispiel 6 beschriebene Versuchsmethode wiederholt.

Die Verbindungen gemäss Beispiel 5 hatten eine gute Wirkung gegen Larven der Spezies Leptinotarsa decemlineata.

Beispiel 8

Wirkung gegen pflanzenschädigende Akarinen : Tetranychus urticae (OP-sensible) und Tetranychus cinnabarinus (OP-tolerant)

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sens) oder Tetranychus cinnabarinus (OP-tol.) belegt. (Die Toleranz bezieht sich auf die Verträglichkeit von Diazinon).

Die so behandelten infestierten Pflanzen wurden mit einer Versuchslösung enthaltend 400 oder 200 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25 °C.

Die Verbindungen gemäss Beispiel 5 zeigten in diesem Versuch eine positive Wirkung gegen Individuen der Spezies Tetranychus urticae und Tetranychus cinnabarinus.

Beispiel 9

Wirkung gegen Rhipicephalus bursa (Imagines und Larven), Amblyomma hebraeum (♀ Imagines, Nymphen und Larven) und Boophilus microplus (Larven — OP-sensible und OP-tolerant).

Als Testobjekte wurden Larven (jeweils ca. 50), Nymphen (jeweils ca. 25) oder Imagines (jeweils ca. 10) von Rhipicephalus bursa, Amblyomma hebraeum und Boophilus microplus verwendet. Die Testtiere wurden für kurze Zeit in eine wässrige Emulsion bzw. Lösung enthaltend 0,1 ; 1,0 ; 10 ; 50 oder 100 ppm der zu prüfenden Verbindung getaucht. Die in Teströhrchen befindlichen Emulsionen bzw. Lösungen wurden dann mit Watte aufgenommen und die benetzten Testtiere in den so kontaminierten Röhrchen belassen.

Eine Auswertung der erzielten Abtötungsrate bei jeder Konzentration erfolgte für Larven nach 3 Tagen und für Nymphen und Imagines nach 14 Tagen.

Die Verbindungen gemäss Beispiel 5 zeigten in diesem Versuch eine gute Wirkung gegen Larven, Nymphen und Imagines der Spezies Rhipicephalus bursa und Amblyomma hebraeum sowie gegen Larven (OP-res. und OP-sens.) der Spezies Boophilus microplus.

Beispiel 10

Wirkung gegen Musca domestica

Je 50 g frisch zubereitetes CSMA-Nährsubstrat für Maden wurde in Becher eingewogen. Von einer 1 Gew.-%igen wässrigen Formulierung des betreffenden Wirkstoffes (dispergierbares Pulver) wurde eine bestimmte Menge auf das in den Bechern befindliche Nährsubstrat pipettiert.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Die Verbindungen gemäss Beispiel 5 zeigten gute Wirkung im obigen Test.

Beispiel 11

Wirkung gegen Aëdes aegypti

Auf die Oberfläche von 150 ml Wasser, das sich in einem Behälter befindet, wurde so viel einer 0,1 %igen Emulsion-Zubereitung des Wirkstoffes pipettiert, dass Konzentrationen von je 10,5 und 1 ppm erhalten wurden. Dann wurde der Behälter mit 30-40 2-tägigen Aëdes-Larven beschickt. Nach 1, 2 und 5 Tagen wurde die Mortalität geprüft.

Die Verbindungen gemäss Beispiel 5 zeigten in diesem Test gute Wirkung gegen Aëdes aegypti.

Beispiel 12

a) Kontaktwirkung auf Eier von

a) Spodoptera littoralis
b) Heliothis virescens
c) Laspeyresia pomonella

Die Eier wurden in einer Lösung bestehend aus 8 ml einer 0,5 %igen Acetonlösung, der zu prüfenden Substanz und 92 ml Wasser während einer Minute getaucht. Nach dem Antrocknen (ca. 1 Std.) wurden die Eier in eine Petrischale gelegt, 4 Tage bei 28 °C gelagert und danach auf Prozent geschlüpfte/nicht geschlüpfte ausgewertet. Die Verbindungen gemäss Beispiel 5 zeigten in diesem Test eine gute Wirkung gegen Eier von Spodoptera littoralis, Heliothis virescens und Laspeyresia pomonella.

b) Gasphasenwirkung auf Eier von Spodoptera littoralis. 2 ml einer 0,5 %igen Acetonlösung wurden in eine Petrischale pipettiert. Ein Aluminiumzylinder mit einem Volumen von 250 ccm wurde in die behandelte Petrischale gestellt. In halber Höhe war der Zylinder durch ein Metallsieb geteilt. Pro Produkt wurden zwei Eigelege auf das Metallsieb gebracht. Der Zylinder wurde mit Cellophanpapier luftdicht abgeschlossen. Die Versuchskontrolle erfolgte nach vier Tagen auf Prozent Schlupf.

Die Verbindungen gemäss Beispiel 5 zeigten eine gute Gasphasenwirkung gegen Eier von Spodoptera littoralis.

**Ansprüche**

1. Cyclopropancarbonsäureester der allgemeinen Formel I

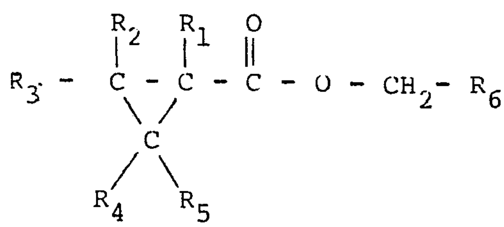

(I)

worin $R_1$, $R_2$, $R_3$, $R_4$ und $R_5$ je ein Wasserstoffatom oder einen Alkylrest, $R_6$ die Reste

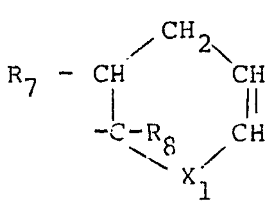

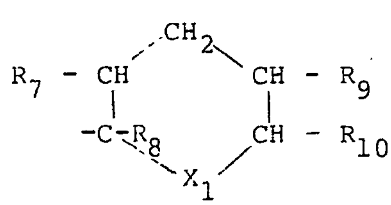

oder

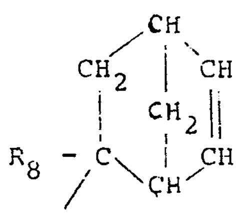

$R_7$ ein Wasserstoffatom, der Methyl- oder Phenyl-Rest, $R_8$ ein Wasserstoffatom, den Methyl- oder den

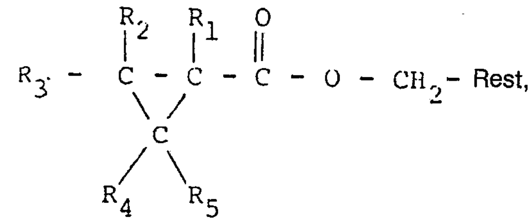

in welchem $R_1$ bis $R_5$ für die oben angegebenen Bedeutungen stehen, $X_1$ für ein Sauerstoffatom, ein Schwefelatom oder für die $—CH_2$-Gruppe steht und $R_9$ und $R_{10}$ je ein Wasserstoffatom oder Halogenatom bedeuten.

2. Verbindungen gemäss Anspruch 1, worin $R_1$ ein Wasserstoffatom oder einen $C_1$-$C_5$-Alkylrest, $R_2$, $R_3$, $R_4$ und $R_5$ je ein Wasserstoffatom oder den Methylrest, $R_6$ die Reste

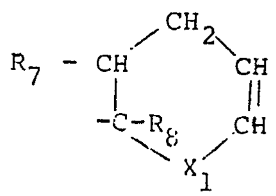

oder

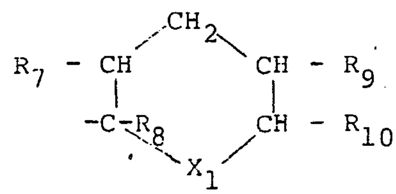

$R_7$ und $R_8$ je ein Wasserstoffatom, $X_1$ ein Sauerstoffatom oder den $—CH_2$-Rest und $R_9$ und $R_{10}$ je ein Wasserstoff-, Chlor- oder Bromatom bedeuten.

3. Verbindungen gemäss Anspruch 1, worin $R_1$, $R_2$, $R_3$, $R_4$ und $R_5$ je ein Wasserstoffatom, $R_6$ die Reste

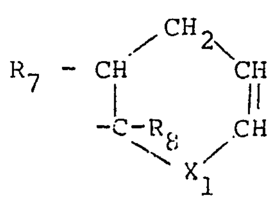

oder

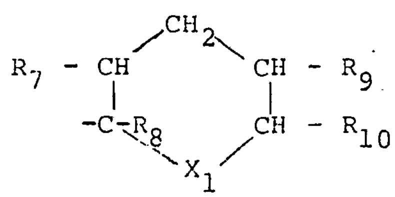

$R_7$ und $R_8$ je ein Wasserstoffatom, $X_1$ ein Sauerstoffatom oder die $—CH_2$-Gruppe und $R_9$ und $R_{10}$ je ein Wasserstoff-, Chlor- oder Bromatom bedeuten.

4. Die Verbindung gemäss Anspruch 3 der Formel

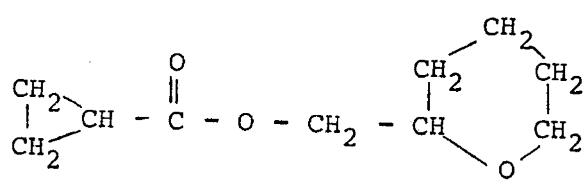

5. Die Verbindung gemäss Anspruch 3 der Formel

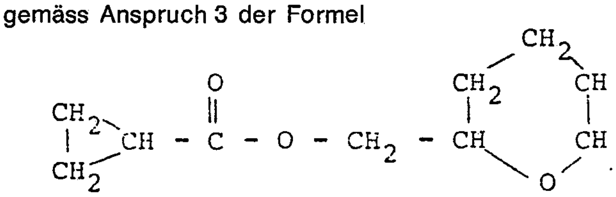

6. Die Verbindung gemäss Anspruch 3 der Formel

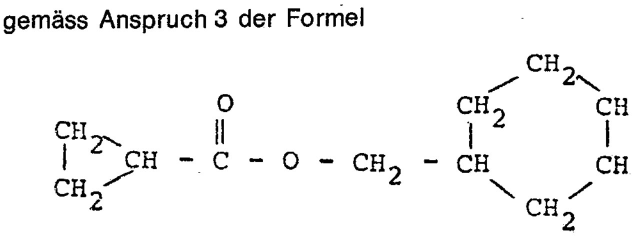

7. Die Verbindung gemäss Anspruch 3 der Formel

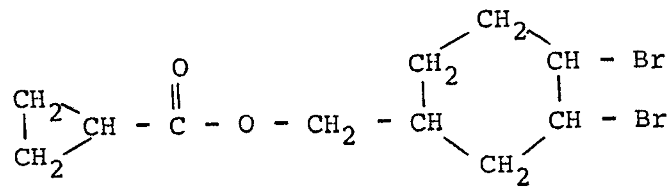

8. Die Verbindung gemäss Anspruch 2 der Formel

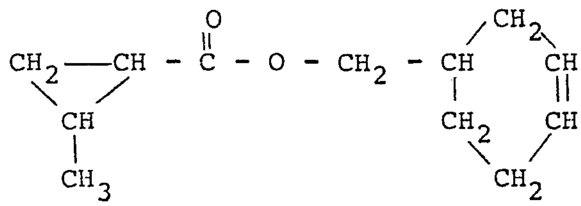

9. Die Verbindung gemäss Anspruch 1 der Formel

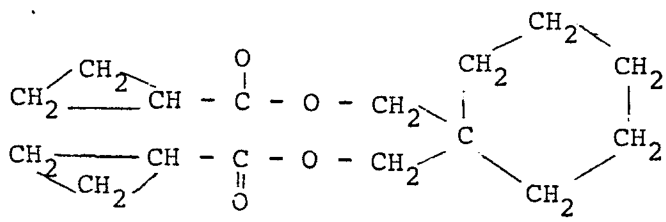

10. Die Verbindung der Formel

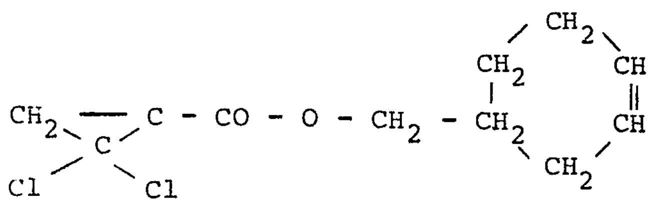

11. Die Verbindung der Formel

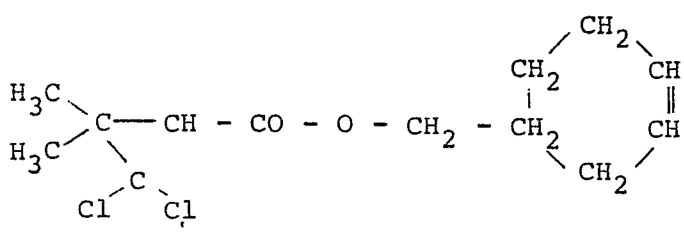

12. Die Verbindung gemäss Anspruch 1 der Formel

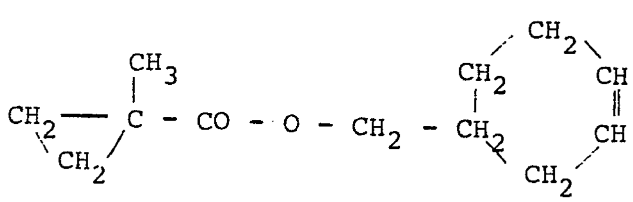

13. Die Verbindung gemäss Anspruch 1 der Formel

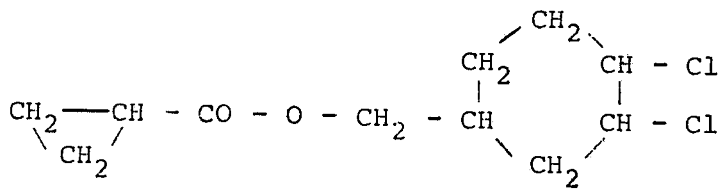

14. Die Verbindung der Formel

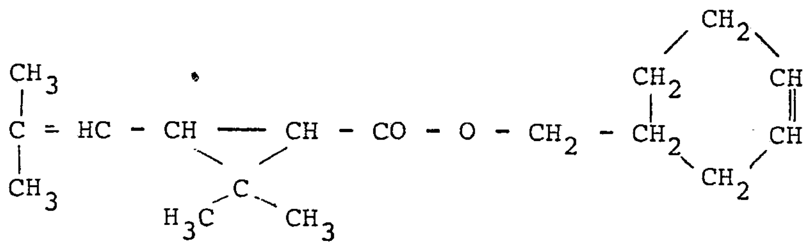

15. Die Verbindung der Formel

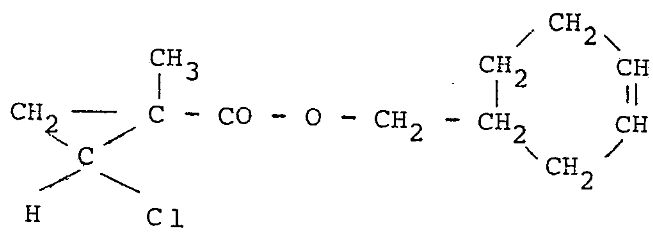

16. Verfahren zur Herstellung von Verbindungen gemäss den Ansprüchen 1, 10, 11, 14 und 15, dadurch gekennzeichnet, dass man eine Verbindung der Formel

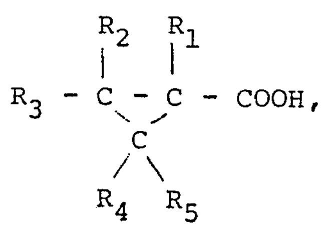

oder eine den Ansprüchen 10, 11, 14 und 15 entsprechende Säure, in Gegenwart eines säurebindenden Mittels mit einer Verbindung der Formel

$$X—CH_2—R_6$$

umsetzt, worin $R_1$ bis $R_6$ die im Anspruch 1 angegebene Bedeutung haben und X für ein Halogenatom steht.

17. Verfahren zur Herstellung von Verbindungen gemäß den Ansprüchen 1, 10, 11, 14 und 15, dadurch gekennzeichnet, daß man eine Verbindung der Formel

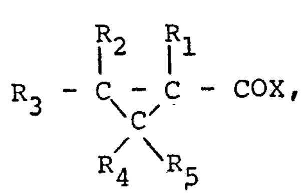

oder ein den Ansprüchen 10, 11, 14 und 15 entsprechendes Halogenid, in Gegenwart eines säurebindenden Mittels mit einer Verbindung der Formel

$$HO—CH_2—R_6$$

umsetzt, worin $R_1$ bis $R_6$ die im Anspruch 1 angegebene Bedeutung haben und X für ein Halogenatom steht.

18. Verfahren zur Herstellung von Verbindungen gemäss den Ansprüchen 1, 10, 11, 14 und 15, dadurch gekennzeichnet, dass man eine Verbindung der Formel

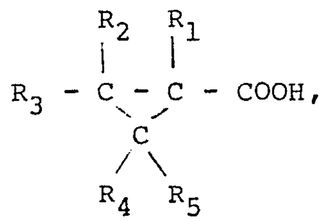

oder eine den Ansprüchen 10, 11, 14 und 15 entsprechende Säure, in Gegenwart eines wasserbindenden Mittels mit einer Verbindung der Formel

$$HO—CH_2—R_6$$

umsetzt, worin $R_1$ bis $R_6$ die im Anspruch 1 angegebene Bedeutung haben.

19. Verfahren zur Herstellung von Verbindungen gemäss den Ansprüchen 1, 10, 11, 14 und 15, dadurch gekennzeichnet, daß man eine Verbindung der Formel

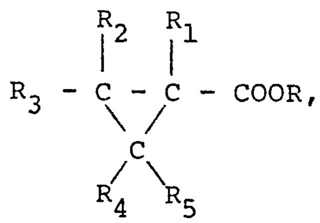

oder ein den Ansprüchen 10, 11, 14 und 15 entsprechender Ester, mit einer Verbindung der Formel

$$HO—CH_2—R_6$$

umsetzt, worin $R_1$ bis $R_6$ die im Anspruch 1 angegebene Bedeutung haben und R für einen $C_1$-$C_4$ Alkylrest steht.

20. Ein Schädlingsbekämpfungsmittel, welches als aktive Komponente eine Verbindung nach den Ansprüchen 1, 10, 11, 14 und 15, sowie geeignete Träger- und/oder andere Zuschlagstoffe enthält.

21. Verwendung einer Verbindung gemäss den Ansprüchen 1, 10, 11, 14 und 15 zur Bekämpfung von verschiedenartigen tierischen und pflanzlichen Schädlingen.

22. Verwendung gemäss Anspruch 21 zur Bekämpfung der Eier, Larven, Nymphen und Imagines von Insekten und Vertretern der Ordnung Akarina.

**Claims**

1. Cyclopropanecarboxylic acid esters corresponding to the general formula I:

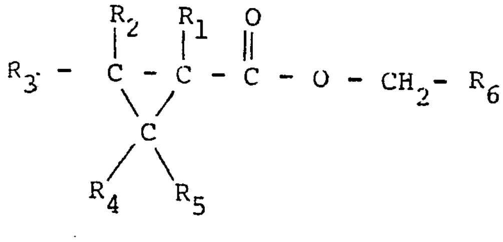

wherein $R_1$, $R_2$, $R_3$, $R_4$ and $R_5$ each represent a hydrogen atom or an alkyl radical, $R_6$ represents the radicals:

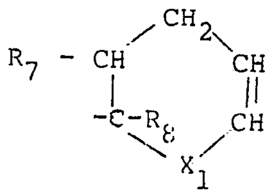 , 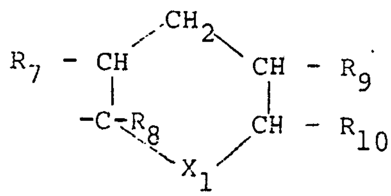

or 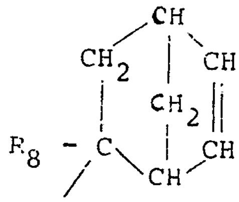 ,

$R_7$ represents a hydrogen atom, the methyl or phenyl radical, $R_8$ represents a hydrogen atom, the methyl or the

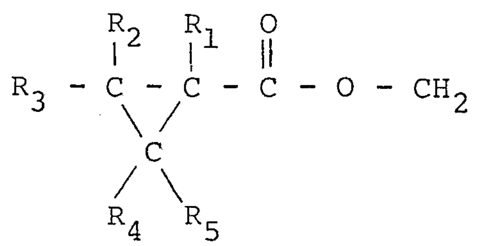 radical

wherein $R_1$ to $R_5$ are as defined above, $X_1$ represents an oxygen atom, a sulphur atom or the $-CH_2$-group, and $R_9$ and $R_{10}$ each represent a hydrogen atom or a halogen atom.

2. Compounds according to claim 1, wherein $R_1$ represents a hydrogen atom a $C_1$-$C_5$ alkyl radical, $R_2$, $R_3$, $R_4$ and $R_5$ each represent a hydrogen atom or the methyl radical, $R_6$ represents the radicals

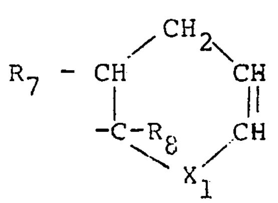 or 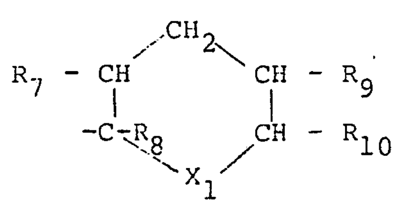

$R_7$ and $R_8$ each represent a hydrogen atom, $X_1$ represents an oxygen atom or the $-CH_2$-radical and $R_9$ and $R_{10}$ each represent a hydrogen, chlorine or bromine atom.

3. Compounds according to claim 1, wherein $R_1$, $R_2$, $R_3$, $R_4$ and $R_5$ each represent a hydrogen atom, $R_6$ represents the radicals

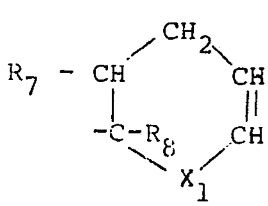 or 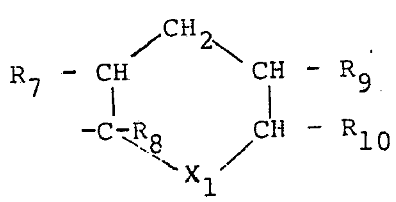

$R_7$ and $R_8$ each represent a hydrogen atom, $X_1$ represents an oxygen atom or the $-CH_2$-group, and $R_9$ and $R_{10}$ each represent a hydrogen, chlorine or bromine atom.

4. The compound according to claim 3 corresponding to the formula :

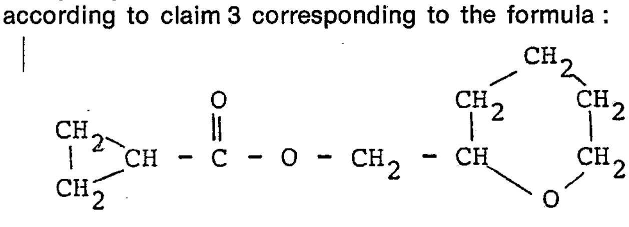

5. The compound according to claim 3 corresponding to the formula :

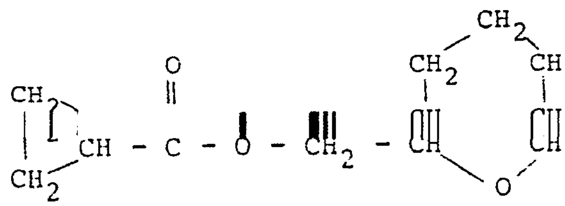

6. The compound according to claim 3 corresponding to the formula :

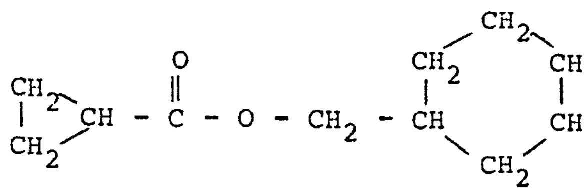

7. The compound according to claim 3 corresponding to the formula :

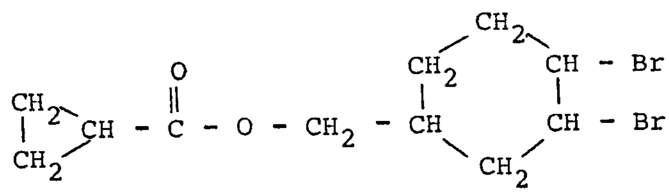

8. The compound according to claim 2 corresponding to the formula :

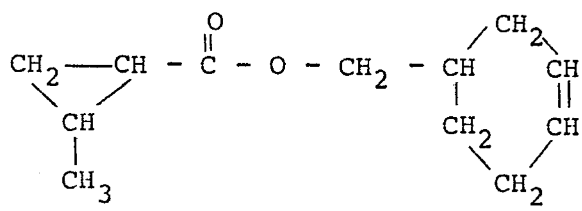

9. The compound according to claim 1 corresponding to the formula :

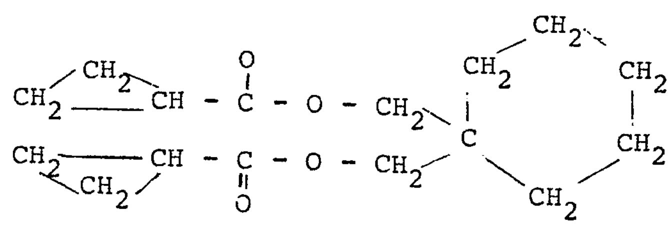

10. The compound corresponding to the formula :

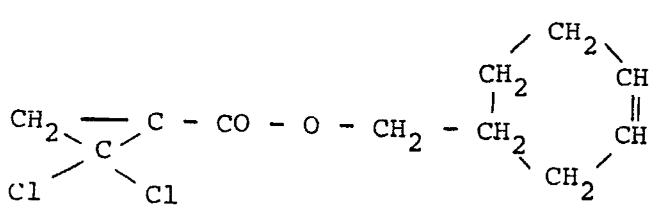

11. The compound corresponding to the formula :

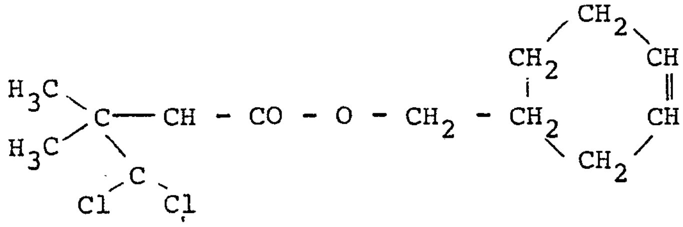

12. The compound according to claim 1 corresponding to the formula :

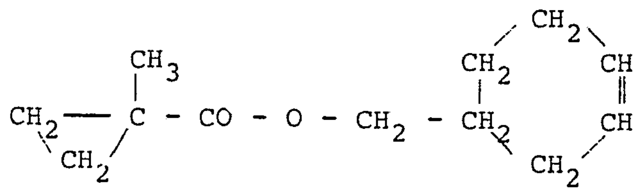

13. The compound according to claim 1 corresponding to the formula :

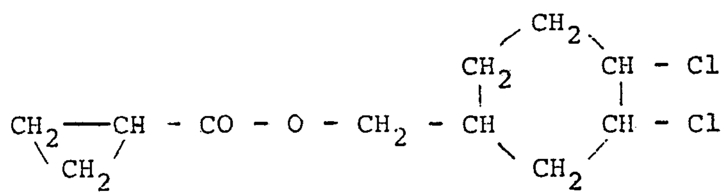

14. The compound corresponding to the formula :

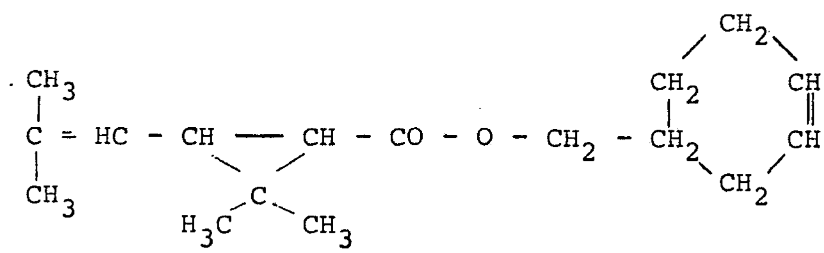

15. The compound corresponding to the formula :

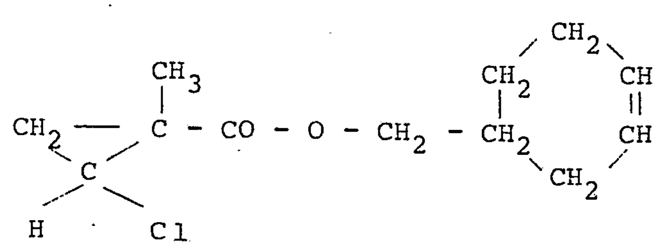

16. A process for the production of compounds according to claims 1, 10, 11, 14 and 15, characterised in that a compound corresponding to the formula :

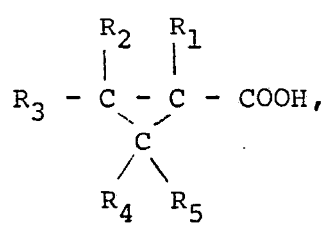

or an acid corresponding to claims 10, 11, 14 and 15 is reacted with a compound corresponding to the formula :

$$X—CH_2—R_6$$

in the presence of an acid-binding agent, wherein $R_1$ to $R_6$ are as defined in claim 1, and X represents a halogen atom.

17. A process for the production of compounds according to claims 1, 10, 11, 14 and 15, characterised in that a compound corresponding to the formula :

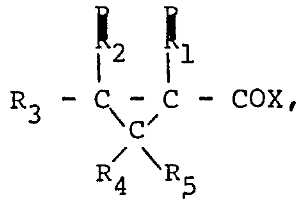

or a halide corresponding to claims 10, 11, 14 and 15, is reacted with a compound corresponding to the formula :

$$HO—CH_2—R_6$$

in the presence of an acid-binding agent, wherein $R_1$ to $R_6$ are as defined in claim 1, and X represents a halogen atom.

18. A process for the production of compounds according to claims 1, 10, 11, 14 and 15, characterised in that a compound corresponding to the formula :

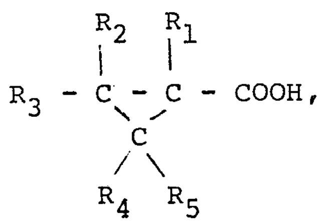

or an acid corresponding to claims 10, 11, 14 and 15 is reacted with a compound corresponding to the formula :

$$HO—CH_2—R_6$$

in the presence of a water-binding agent, wherein $R_1$ to $R_6$ are as defined in claim 1.

19. A process for the production of compounds according to claims 1, 10, 11, 14 and 15, characterised in that a compound corresponding to the formula :

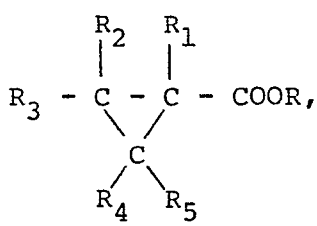

or an ester corresponding to claims 10, 11, 14 and 15, is reacted with a compound corresponding to the formula :

$$HO—CH_2—R_6$$

wherein $R_1$ to $R_6$ are as defined in claim 1, and R represents a $C_1$-$C_4$ alkyl radical.

20. A pesticide which contains as an active component a compound according to claims 1, 10, 11, 14 and 15, and suitable carriers and/or other additives.

21. The use of a compound according to claims 1, 10, 11, 14 and 15 to control various animal and plant pests.

22. The use according to claim 21 to control the eggs, larvae, nymphs and imagines of insects and members of the Acarina order.

**Revendications**

1. Ester de l'acide cyclopropanecarboxylique de formule générale I :

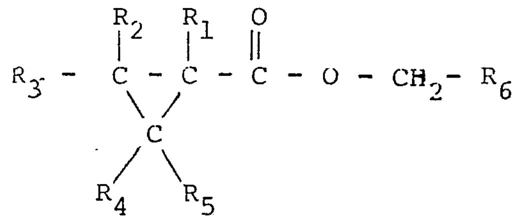

(I)

où $R_1$, $R_2$, $R_3$, $R_4$ et $R_5$ représentent chacun un atome d'hydrogène ou un reste alcoyle, $R_6$, les restes

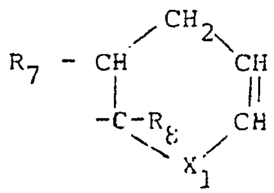

,

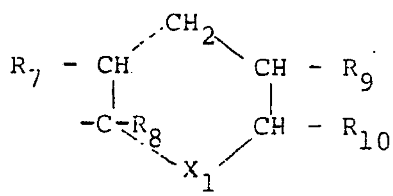

ou

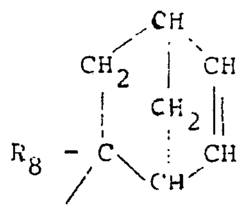

,

$R_7$ représente un atome d'hydrogène, le reste méthyle ou phényle, $R_8$, un atome d'hydrogène, les restes méthyle ou

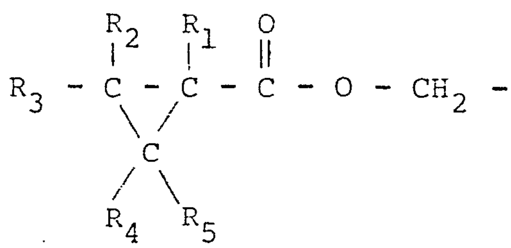

où $R_1$ à $R_5$ ont la signification indiquée plus haut, $X_1$ représente un atome d'oxygène, un atome de soufre, ou le groupe —$CH_2$—, et $R_9$ et $R_{10}$ représentent chacun un atome d'hydrogène ou un atome d'halogène.

2. Composés suivant la revendication 1, où $R_1$ représente un atome d'hydrogène ou un reste alcoyle en $C_1$ à $C_5$, $R_2$, $R_3$, $R_4$ et $R_5$ représentent chacun un atome d'hydrogène ou le reste méthyle, $R_6$, les restes

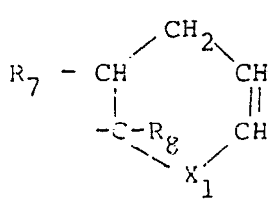

ou

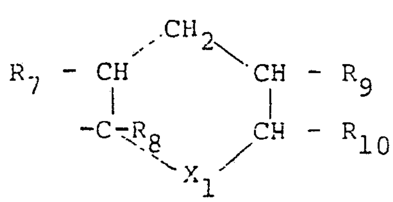

$R_7$ et $R_8$ représentent chacun un atome d'hydrogène, $X_1$ un atome d'oxygène ou le reste —$CH_2$—, et $R_9$ et $R_{10}$, chacun un atome d'hydrogène, de chlore, ou de brome.

3. Composés suivant la revendication 1, où $R_1$, $R_2$, $R_3$, $R_4$ et $R_5$ représentent chacun un atome d'hydrogène, $R_6$ les restes :

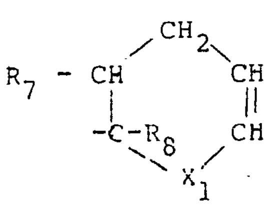

ou

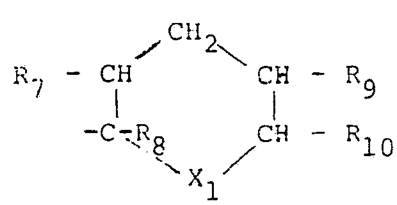

$R_7$ et $R_8$, chacun un atome d'hydrogène, $X_1$ un atome d'oxygène ou le groupe $—CH_2—$, et $R_9$ et $R_{10}$, chacun un atome d'hydrogène, de chlore, ou de brome.

4. Composé suivant la revendication 3 de formule :

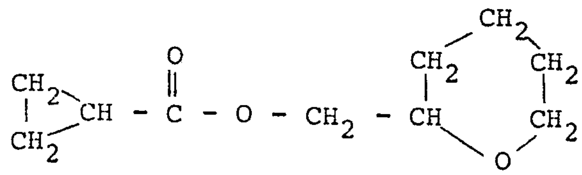

5. Composé suivant la revendication 3 de formule :

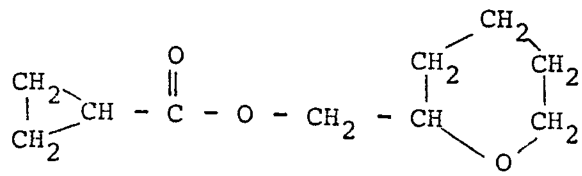

6. Composé suivant la revendication 3 de formule :

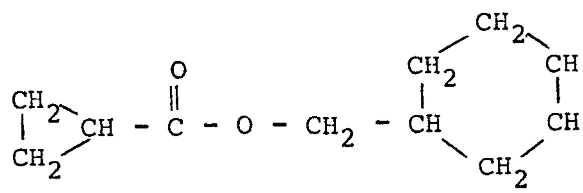

7. Composé suivant la revendication 3 de formule :

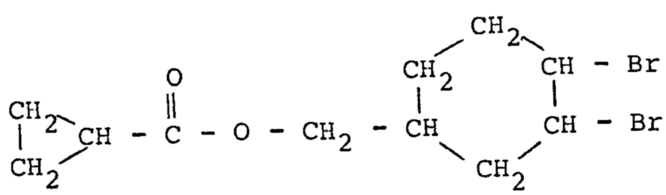

8. Composé suivant la revendication 2 de formule :

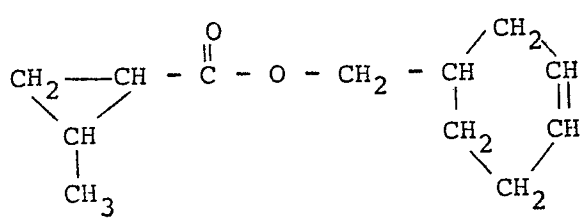

9. Composé suivant la revendication 1 de formule :

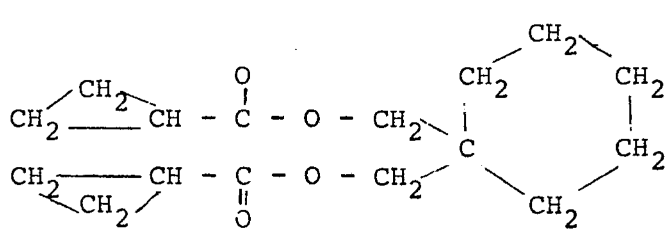

10. Le composé de formule :

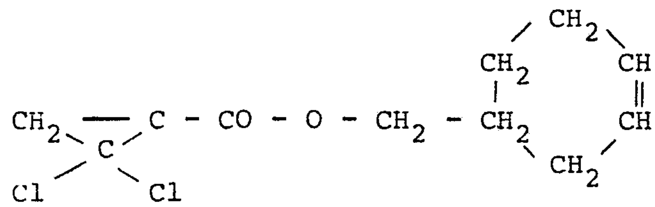

11. Le composé de formule :

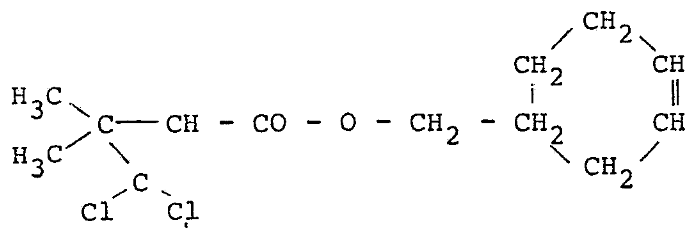

12. Composé suivant la revendication 1 de formule :

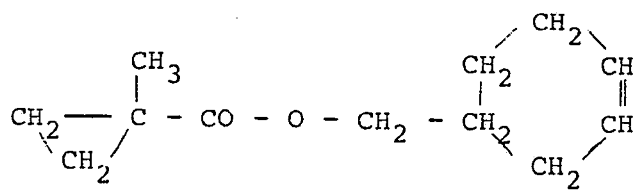

13. Composant suivant la revendication 1 de formule :

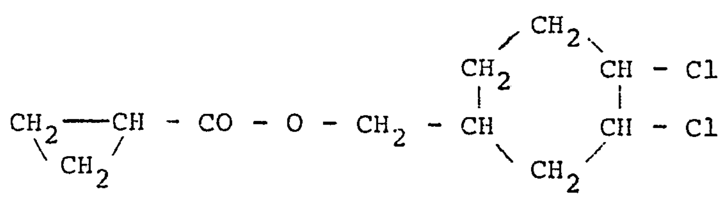

14. Composé de formule :

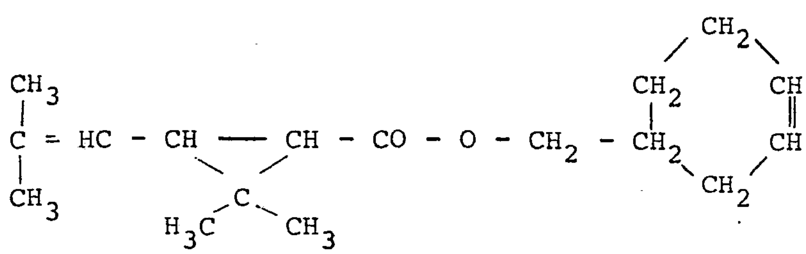

15. Composé de formule :

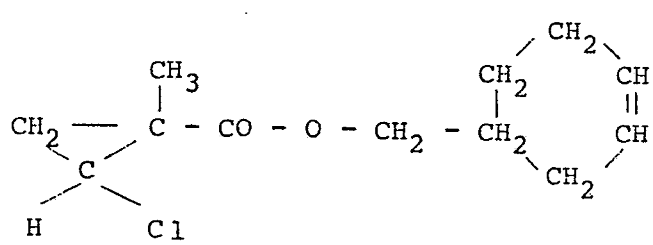

16. Procédé pour la fabrication de composés suivant l'une quelconque des revendications 1, 10, 11, 14 et 15, caractérisé en ce que l'on fait réagir un composé de formule :

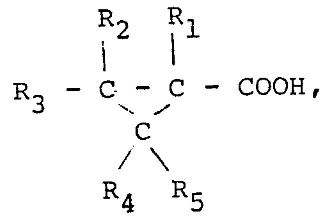

ou un des acides correspondant aux revendications 10, 11, 14 et 15, en présence d'un agent se combinant avec l'acide, avec un composé de formule :

$$X—CH_2—R_6$$

où $R_1$ et $R_6$ ont les significations indiquées dans la revendication 1 et X représente un halogène.

17. Procédé pour la fabrication de composés suivant l'une quelconque des revendications 1, 10, 11, 14 et 15, caractérisé en ce que l'on fait réagir un composé de formule :

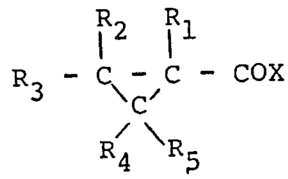

ou un halogénure correspondant à une des revendications 10, 11, 14 et 15, en présence d'un agent se combinant avec l'acide, avec un composé de formule :

$$HO—CH_2—R_6$$

où $R_1$ à $R_6$ ont la même signification que dans la revendication 1 et X représente un atome d'halogène.

18. Procédé pour la fabrication de composés suivant l'une quelconque des revendications 1, 10, 11, 14 et 15, caractérisé en ce que l'on fait réagir un composé de formule :

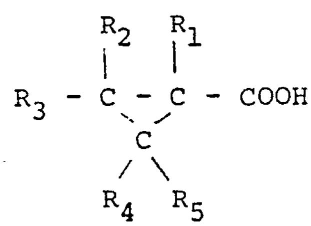

ou un acide correspondant aux revendications 10, 11, 14 et 15, en présence d'un agent se combinant avec l'eau, avec un composé répondant à la formule :

$$HO—CH_2—R_6$$

$R_1$ à $R_6$ ayant les significations indiquées dans la revendication 1.

19. Composé pour la fabrication de composés suivant l'une quelconque des revendications 1, 10, 11, 14 et 15, caractérisé en ce que l'on fait réagir un composé de formule :

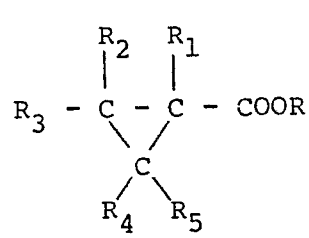

ou un ester correspondant aux revendications 10, 11, 14 et 15, avec un composé de formule :

$$HO—CH_2—R_6$$

où $R_1$ à $R_5$ ont la signification indiquée dans la revendication 1, et $R_6$ représente un reste alcoyle en $C_1$ à $C_4$.

20. Agent pour la lutte contre les insectes nuisibles qui contient, comme composé actif, un composé suivant l'une quelconque des revendications 1, 10, 11, 14 et 15, ainsi que les supports et/ou produits auxiliaires appropriés.

21. Utilisation d'un composé suivant l'une quelconque des revendications 1, 10, 11, 14 et 15, pour lutter contre des organismes nuisibles, animaux et végétaux, de différents types.

22. Utilisation suivant la revendication 21 pour la lutte contre les œufs, nymphes, larves et imagos d'insectes et de représentants de l'ordre Akarina.